# EUROPEAN PATENT APPLICATION

(11) **EP 4 438 079 A1**
(43) Date of publication of application: **02.10.2024**
(21) Application number: 23165785.9
(22) Date of filing: 31.03.2023
(51) Int. Cl.: A61M 5/158, A61B 90/90, A61B 90/92, A61M 25/00, A61M 39/10, A61M 39/12

(54) **CONNECTOR FOR CATHETER AND LABEL PLATE THEREFOR**

(71) Applicant: BIOTRONIK AG, 8180 Bülach (CH)
(72) Inventor: von Orelli, Luc, 8003 Zürich (CH); Taormina, Matteo, 8193 Eglisau (CH)
(74) Representative: Biotronik Corporate Services SE

(57) **Abstract**

The invention refers to a catheter connector (1, 31) and a label plate (10, 40, 50, 70, 90, 110, 110a) for such connector (1, 31), wherein the label plate comprises a label plate body and at least one fixation element (16, 46, 66, 76, 86, 96, 106), wherein the label plate is configured to be fixed to an outer shell surface of the catheter connector by the at least one fixation element and wherein the shape of a bottom side or inner surface of at least a segment of the label plate body corresponds to the shape of an outer surface of at least a section of the catheter connector.

## Description

The invention relates generally to marking of catheters and its connectors.

Catheters are medical devices that comprise an elongated, usually flexible shaft made of medical grade materials and serve a broad range of functions. Normally, they are inserted into a patient's body for diagnosis and/or treatment. For example, the catheter may be inserted into a body cavity, duct, or vessel, brain, skin or adipose tissue. Functionally, a catheter may allow drainage, administration of fluids or gases, access by surgical instruments, or sampling of compounds from the patient's body. One important catheter type is a balloon catheter that has an inflatable balloon at its distal end and is used to expand/deploy a stent or similar implant or to enlarge a narrow opening or passage within the patient's body. Such balloon catheter may be used in angioplasty or balloon septostomy, e.g. via cardiac catheterization, tuboplasty via uterine catheterization or pyeloplasty.

In order to provide its specific function, in many cases the catheter needs to be connected to a specific device (e.g. inflation device) or reservoir by at least one male or female connector (fitting) which is typically provided at or near a proximal end of the elongated shaft. For correct usage the catheter's connector is connected to the specific device or reservoir that fits to the specific function and size of the catheter and/or connector. To assist the health care professional (HCP) in choosing the right catheter (e.g. among different catheters in use), the connector may comprise a specific marking that is provided, for example, at a handle-like section or on a surface of the connector. However, usually a catheter connector is transparent allowing to observe the interior of the connector. Additionally, the connector often has a small size o that a specific marking is difficult to read. In case there is the desire to use a colored marking with the connector, such colorizing additive sometimes changes the properties of the plastic material often used for such connector. This forces a costly admission process when the used material is changed and increases the costs due to extensive warehousing for catheters having different colors.

A less costly solution for a better readable marking of catheters and catheter connectors is therefore required that allows an easy and fast identification of the catheter or its connector.

The above object is solved by a label plate having the features of claim 1, by a system comprising a catheter connector and such label plate having the features of claim 11 as well as by a system comprising a catheter and such label plate having the features of claim 12.

In particular, the above object is solved by a label plate for a catheter connector, wherein the label plate comprises a label plate body and at least one fixation element, wherein the label plate is configured to be fixed to the outer shell surface of the catheter connector by the at least one fixation element and wherein the shape of a bottom side or inner surface of at least a segment of the label plate body corresponds to the shape of an outer surface of at least a section of the catheter connector.

The label plate according to the invention comprises at least one fixation element and a label plate body. The at least one fixation element is used to firmly or releasably attach the label plate to the catheter connector, namely to its outer surface (a firm attachment of the label plate to the catheter connector may be a firm-bond in the sense of a material bond or a firm form or force bond which is only releasable by destruction of the label plate, in particular the fixation element, or the catheter connector). This is important, because the label plate is used to mark the connector and/or the catheter without changing the composition of the connector, for example, to show the type and size of the connector and/or catheter. For that, the marking can be provided and represented by the properties of the label plate body itself (its color, form, size, surface structure or similar) and/or may be printed or written to the surface of the label plate body. The label plate body may comprise or consist of a material that is different from the material of the connector. Unlike the material of the connector, which is adjusted and chosen to its specific medical purpose, the material of the label plate body may be chosen and adjusted to realize a better readable marking (e.g. by the color of the material or by being better markable by a marking, i.e. writing, method). The label plate may further be stored separately from the catheter and its connector and may be assigned and fixed to the outer shell surface of the respective connector shortly prior to sterilization (during manufacturing), only. For a firm-bond in the sense of a material bond, the fixation element may, for example, be glued or laser welded to the catheter connector.

That the shape of a bottom side or inner surface of at least a segment of the label plate body corresponds to the shape of an outer surface of at least a section of the catheter connector (e.g. of the handle-like section) means that the side or surface of the label plate body that is configured to abut the outer surface of the connector corresponds to the shape of this surface. Accordingly, the correct position of the label plate at the connector is easily and quickly adopted. Further, thereby the volume occupied by the label plate at the connector is small. Additionally, the at least one fixation element may correspond to the shape of the outer surface of the catheter connector, as well.

The term "connector" as it is used in the present description refers to a component provided at the proximal end of the catheter. The connector may comprise a plurality of sections, for example the actual connector section (i.e. in the literal sense) where the catheter is connected with the connector's counterpart provided by a device or a reservoir to be connected with the catheter, a handle-like section where the user (HCP) grips the connector to attach the connector to its counterpart and a transition section where the connector is attached to the elongated and flexible catheter shaft. The connector section in the narrower sense and the handle-like section may be rigid, while the transition section may have an intermediate flexibility between the rigidity of the rigid sections and the flexibility of the adjacent catheter shaft. The label plate may be fixed to at least one of the above sections of the connector. In one embodiment, the label plate may be fixed to the handle-like section. In one embodiment, the connector section in the narrower sense is the most proximal section and adjacent to the handle-like section and the transition section is the most distal section of the connector (in the broad sense). The connector comprises at least one opening extending from the proximal end to the distal end that goes through.

The label plate allows to provide a marking to the catheter and/or its connector in an easy and cost-effective way. A further advantage is that the label plate may be configured such that the area where a making is provided can be designed much greater than the underlying area of the connector. Accordingly, the label plate can increase the surface available for marking so that the marking may contain more information for the HCP. The marking may include numbers, letters and picture-like marking such as pictograms or barcodes, RFID tags as well as trademark signs and similar marking. For example, the marking may contain (e.g. in case the catheter is a balloon catheter) the nominal pressure (NB), the rated burst pressure (RBP), and/or the maximum allowed diameter (MAD). The label plate (in manufacturing) may be stored separately from the catheter or the connector and may consist of a nontransparent material that allows marking with a greater contrast that is easier to read. In particular, a coloration or specific form of the label plate allows fast identification of the correct catheter and/or connector in a stressful clinic environment. A further advantage of such label plate is that it's look, and feel may be adapted to the user's, authorities' and market's requirements fast and without any need (or reduced requirements for passing regulatory admission process) as the label plate does not change the interior construction or material of the connector. It is just fixed to the outer surface of the connector.

In one embodiment, the label plate or the shape of the label plate is configured such that it allows exposure of a central portion of the catheter connector that extends along a longitudinal axis section of the catheter connector and/or a lateral axis section of the catheter connector. In some embodiments, the label plate body may comprise a through-hole and/or a recess, wherein the through-hole and/or the recess are configured to be arranged above the central portion of the catheter connector and to extend along the longitudinal axis section of the catheter connector and/or the lateral axis section of the catheter connector in a state fully mounted to the catheter connector. In addition or alternatively, the shape of the label plate may be of a dimension that at least part of the central portion of the catheter connector that extends along a longitudinal axis section of the catheter connector and/or the lateral axis section of the catheter connector is not covered by the label plate when fixed to the outer shell surface of the catheter connector. In addition or alternatively, the label plate may comprise a window section comprising and/or being made of a transparent material and being arranged at the label plate so that it reveals the central portion of the catheter connector that extends along a longitudinal axis section of the catheter connector and/or a lateral axis section of the catheter connector when the label plate is fixed to the catheter connector.

The label plate or the shape of the label plate is configured to allow exposure of a central portion of the catheter connector that extends a longitudinal axis section of the catheter connector to allow the HCP to observe the interior of the connector in the area of the connector's longitudinal axis. Thereby, the HCP may control whether the connector/catheter operates correctly during the patient's treatment or diagnosis. Such shape may provide a window-like or slit-like opening through which the HCP may observe the central portion of the catheter. The longitudinal axis/direction of the connector is the extension of the longitudinal axis/direction of the catheter shaft. A lateral axis of the connector is any axis which forms an angle unequal to 0° with the longitudinal axis of the catheter connector. The central portion is a portion of the connector that extends along and parallel the longitudinal axis/direction, respectively, the lateral axis and the through opening. This portion may include only part of the width (greater extension perpendicular to the longitudinal axis) of the connector but the central part of the width so that the HCP may observe the fluid or other material running along the through opening of the connector. The central portion may comprise only a part of the full length of the connector (extension along the longitudinal axis).

In one embodiment, the at least one segment has a U-shape in a state fully mounted to the catheter connector, wherein the U-shape provides an intermediate space (slit-like opening) to allow exposure of a central portion of the catheter connector that extends along the longitudinal axis section of the catheter connector. In another embodiment, the at least one segment has a frame-like shape in a state fully mounted to the catheter connector, wherein the frame-like shape provides window-like opening to allow exposure of a central portion of the catheter connector that extends along a longitudinal axis section of the catheter connector. The intermediate space or window-like opening is an open space located between the two legs of the U-shape. In one embodiment, the connector's central area along its longitudinal axis is covered by the label plate body only in or near the base section of the "U" or "frame" - where the two legs or sides of the frame are connected.

In one embodiment, the U-shaped segment is formed by at least one first plate-like leg, at least one second plate-like leg and at least one plate-like web connecting one of the at least one first leg and one of the at least one second leg. In this embodiment the open intermediate space is formed between the first and the second leg, wherein the web may cover a section of the longitudinal axis of the connector when it is fully mounted to the connector. In one embodiment, the web is located on the same side of the connector as the first and second plate-like legs. In an alternative embodiment, the web is located on the opposite side of the connector as the first and second legs.

The fixing of the label plate to the connector is provided by at least one fixation element. The fixation element may be located separate from and/or adjacent to the label plate body and/or may be integrated within the label plate body. In one embodiment, the at least one fixation element and/or the label plate body is configured such that it provides a form-fitting connection, a force-fitting connection and/or a firmly bonded connection with the catheter connector. For example, the label plate body may comprise at least two protrusions that clamp onto the outer rim of the connector forming a snap-fit connection. Alternatively, such protrusions may interact with at least two protrusions at the opposite side of the label plate body forming a snap-fit connection so that the connector is arranged between an upper segment of the label plate body and a lower segment of the label plate body. Additionally, or alternatively, the label plate body may comprise an adhesive at one side or at the fixation element that is used to attach the label plate to the surface of the connector.

In one embodiment, a material located at the upper side or outer surface of the label plate body (i.e. a surface that points away from the connector) is writable or printable. Accordingly, the desired marking may be provided in an easy and cost-effective way. For example, the material located at the upper side or outer surface of the label plate body may comprise laser sensitive particles or pigments for laser marking. Alternatively, inkjet printing may be used for printing the desired information to the label plate body. In this case, the material at the outer surface or outer side of the label plate body is configured such that ink or toner used for inkjet printing may adhere to the surface/side. In one embodiment, the printing ink or toner may be additionally adhered to the surface of the label plate by a cover layer covering the printed marking with a thermal or curable material that may be toughened or hardened after printing by a thermal or UV treatment. Alternatively, the printing ink or toner itself may be cured by thermal or UV treatment.

In one embodiment, the label plate body comprises an appendage or similar projection providing a secondary function, wherein the appendage (e.g. a projection) is, for example, formed as a guide wire insertion tool. The guide wire insertion tool may ease the introduction of the guide wire into a catheter tube as explained in more detail below. The appendage may be removed from the label plate prior the secondary function is started or executed. For that, a predetermined breaking point may be provided between the label plate and the appendage for easy removal.

In one embodiment, the label plate body comprises two opposite segments, wherein at least one segment of the two segments comprises the bottom side or inner surface with the shape which corresponds to the shape of the outer surface of at least a section of the catheter connector, e.g. a U-shape, wherein both segments are connected by a hinge. Such hinge allows correct relative movement (folding) of two opposite segments prior the label plate adopts its fully mounted state, respectively, is connected to the catheter connector. In one embodiment, the catheter connector is located between the first segment and the opposite second segment in the fully mounted state. In one embodiment, both, the first and the second segment have a U-shape.

In one embodiment, the at least one fixation element and/or the label plate body comprises a predetermined breaking point. As indicated above, such predetermined breaking point may allow removal of an appendage with a secondary function from the label plate. Alternatively or additionally, such breaking point may indicate to the user if the label plate has been removed from the connector without authorization.

In one embodiment, an RFID tag is embedded within the label plate body material. The RFID tag may comprise a microchip for storing and processing information and production of the RF signal, an antenna for receiving and transmitting the RF signal and a substrate.

The above object is further solved by a system comprising a catheter connector and a label plate as described above, wherein the shape of a bottom side or inner surface of the label plate body corresponds to the shape of the outer surface of a section of the catheter connector.

The connector is a connector in a broader sense as explained above. The system has the advantages that are described above with regard to the label plate. It is therefore referred to the above discussion.

The above object is further solved by a system comprising a catheter having an elongated, flexible shaft and a connector at the proximal end of the shaft, wherein the system further comprises a label plate as described above, wherein the shape of a bottom side or inner surface of the label plate body corresponds to the shape of the outer surface of a section of the catheter connector. The connector is a connector in a broader sense as explained above. The system has the advantages that are described above with regard to the label plate. It is therefore referred to the above discussion.

In one embodiment of both above systems, the catheter connector section (in the narrower sense) of the connector is a male or female Luer connector. Such connector is often used for attachment in the medical field for providing leak-free connections. The Luer connector is used to form a Luer taper together with its respective mating connector.

In one embodiment of the system comprising the catheter, the catheter is a balloon catheter and comprises an inflatable balloon at the distal end of the catheter shaft.

With respect to all above embodiments, the connector (in the broader sense) may comprise or consist of a material of the group of thermoplastics, the label plate may also comprise or consist of a material of the group of thermoplastics. Further the label plate or each segment of the label plate may have a thickness of about 0.2 to 5 mm, preferably of about 0.2 to 2 mm. The surface area for writing / printing at the label plate may be greater than 5mm x 15mm = 75mm². The label plate may be produced by injection moulding or 3D printing.

The catheter may comprise, for example a single or double connector at its proximal end, an inflatable/deflatable balloon at its distal end and a flexible, elongated shaft in between having a longitudinal axis. Within a distal section of the shaft, the tube may comprise two lumen wherein a first lumen is the inflation lumen that is in fluid communication with the balloon and a guide wire lumen. The guide wire lumen may be provided by an inner tubular member whereas the inflation lumen may be provided by an outer tubular member. The guide wire may run along an outer surface of the catheter shaft within a proximal section of the catheter shaft.

The present invention will now be described in further detail with reference to the accompanying schematic drawings, wherein
- Fig. 1: shows a first embodiment of a label plate and a first embodiment of a catheter in a perspective side view during manufacturing,
- Fig. 2: depicts the first embodiment of a label plate fixed to a second embodiment of a catheter in a perspective side view in a fully mounted state,
- Fig. 3: shows the system of Fig. 2 in a different perspective side view in a fully mounted state,
- Fig. 4: shows a second embodiment of a label plate and the catheter of Fig. 1 in a perspective side view during manufacturing,
- Fig. 5: depicts a third embodiment of a label plate and the catheter of Fig. 1 in a perspective side view during manufacturing,
- Fig. 6.: depicts a fourth embodiment of a label plate and the catheter of Fig. 1 in a perspective side view during manufacturing,
- Fig. 7: depicts a fifth embodiment of a label plate and the catheter of Fig. 1 in a perspective side view during manufacturing,
- Fig. 8: shows a sixth embodiment of a label plate and the catheter of Fig. 2 in a perspective side view in a fully mounted state,
- Fig. 9: depicts a seventh embodiment of a label plate and the catheter of Fig. 2 in a side view in a fully mounted state, and
- Fig. 10 - 12: depict the usage of a guide wire insertion tool for guide wire introduction in side views.

In the following, the invention is described with regard to balloon catheters. Generally, the invention may also be applied to other catheter types so that the following examples are not intended to limit the scope of the disclosed subject matter in any manner, in particular not to balloon catheters.

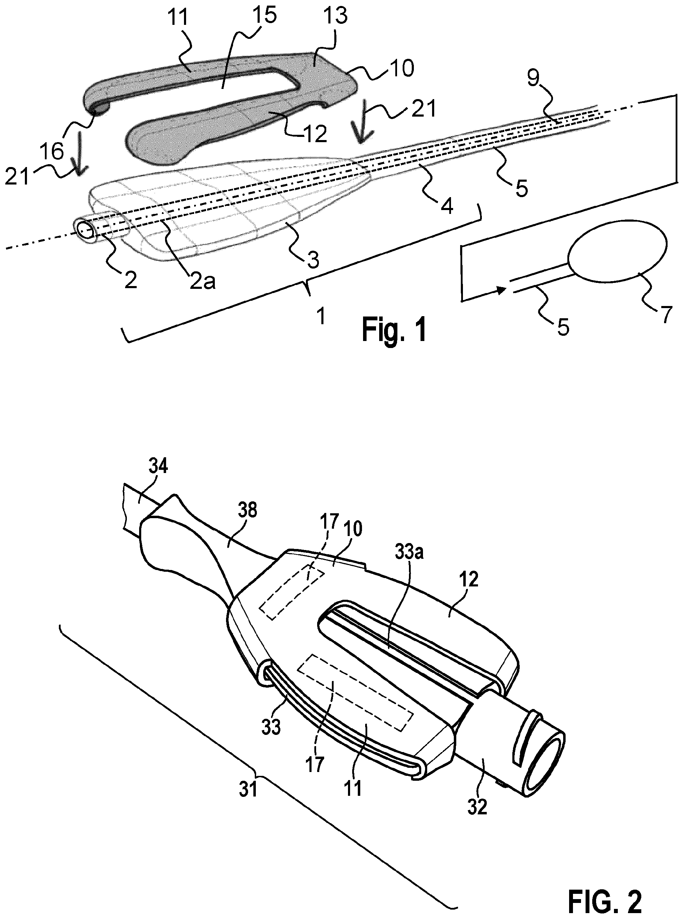

Fig. 1 shows a balloon catheter comprising a tubular shaft 5 that is connected at its proximal end to a connector 1 and at its distal end to a balloon 7. The tubular shaft 5 and the connector 1 have a longitudinal axis 9. The shaft 5 comprises an inflation lumen, wherein the balloon 7 is in fluid communication with the inflation lumen. The inflation lumen extends from the connector 1 to the balloon 7. At the distal end the shaft 5 comprises a guide wire lumen that runs parallel to the inflation lumen and through the balloon 7. A guide wire extends through this guide wire lumen during operation of the catheter for guiding the catheter to the treatment/diagnosis location.

The connector 1 comprises a connector section 2 at its distal end and a transition section 4 at its proximal end. A flat handle-like section 3 is provided in longitudinal direction between the connector section 2 with, e.g. a male Luer connector and the transition section 4. The transition section 4 forms the transition to the flexible shaft 5 and a fluid tight connection to the shaft 5 and is more flexible than the rigid connector section 2 and handle-like section 3. The handle-like section 3 can be gripped by the HCP to connect the catheter to a pre-determined device or reservoir having a connector counterpart (e.g. a female Luer connector). The connector 1 is formed of transparent plastic material so that the HCP may observe the fluid running a long an opening 2a going through the connector 1.

The system further comprises a label plate 10 with a U-shaped body having a first plate-like leg 11, a second plate-like leg 12 and a plate-like web 13 connecting the first leg 11 and the second leg 12 at their distal ends. Between the first leg 11 and the second leg 12 there is an intermediate space 15 that allows exposure of a central portion of the connector 1 located at and around the longitudinal axis 9 of the connector 1. Each of the first leg 11 and the second leg 12 comprises a protrusion at its proximal end that is bent such that it provides a form-fitting connection with the handle-like section 3 of the connector 1. Additionally, similar protrusions are provided at the ends of the web 13 extending transverse to the longitudinal axis 9. The label plate 10 may be firmly attached to the section 3 of the connector 1 by clamping (see arrows 21) using the protrusions 16 that engage around the rim of section 3 of the connector 1 in the fully mounted state.

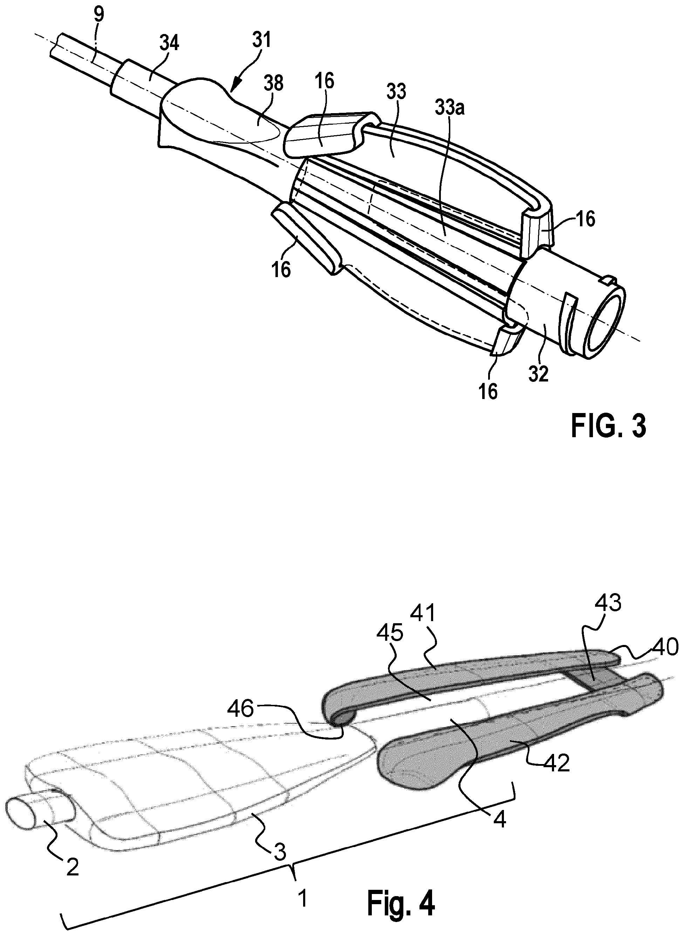

A similar attachment and label plate 10 is shown in Fig. 2 and 3 in a fully mounted state. The connector 31 of the catheter is different from the one depicted in Fig. 1 in that the connector 31 comprises a fourth section between the transition section 34 and the first handle-like section 33 in longitudinal direction - namely a second handle-like section 38. The second handle-like section 38 is smaller than the first handle-like section 33. The upper flat surfaces of the first and the second handle-like sections 33, 38 are twisted by approximately 90 °. The connector 31 further comprises a connector section 32 and a transition section 24 similar to the catheter connector 1 shown in Fig. 1. In Fig. 2 and 3 it is clearly shown that the intermediate space 15 of the U-shaped label plate body exposes a central portion 33a of the first handle-like section 33 of the connector 31 for observation of the through opening (analogous to opening 2a of connector 1 shown in Fig. 1). Further, Fig. 3 depicts how the protrusions 16 encompass the rim of the first handle-like section 33. As one can derive from Fig. 1 to 3, the lower side of the label plate 10 abutting to the upper surface of the handle-like section 3, 33 has a shape that corresponds to the upper surface of the handle-like section 3, 33. Thereby, the thickness of the respective handle-like section 3, 33 is only slightly increased compared with the thickness of the handle-like section 3, 33 without label plate 10.

Fig. 2 further shows that label plate 10 has a color different from the color of the connector 31. Further, as it is not transparent, the marking 17 has a clear contrast with regard to the label plate body and thereby easy readable.

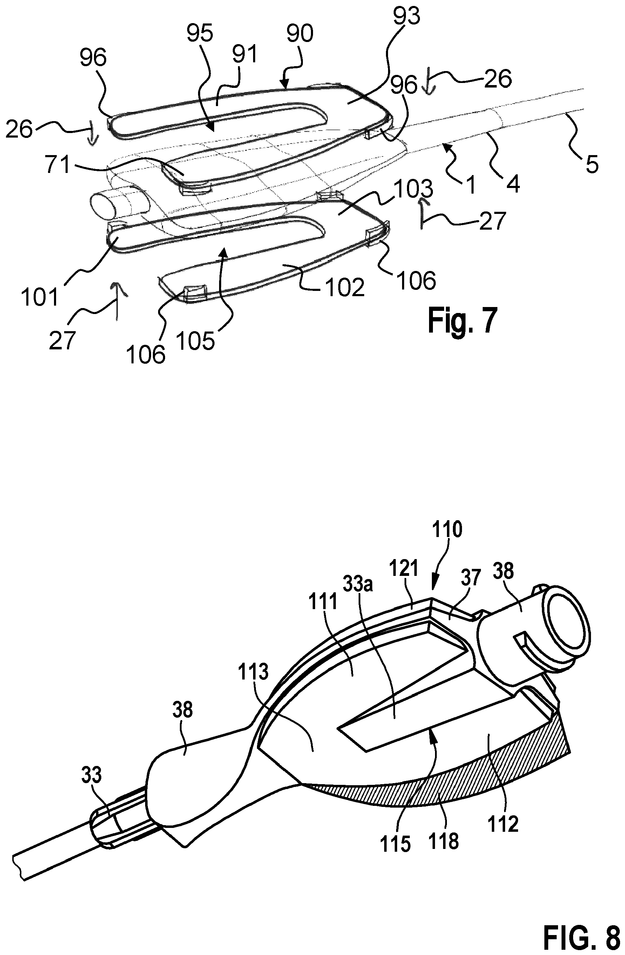

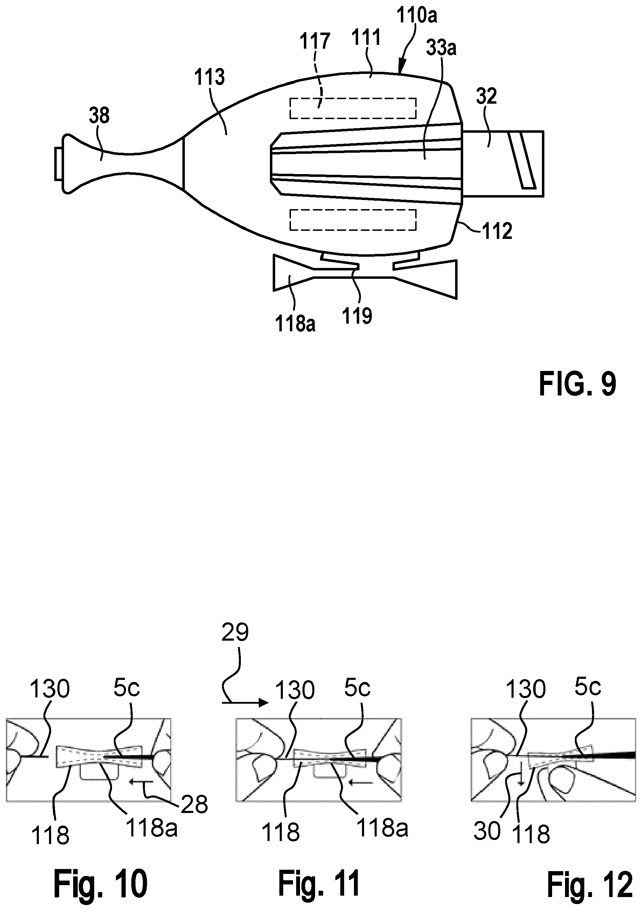

The embodiments of the system shown in Fig. 4 to 7 refer to a catheter and connector 1 as shown in Fig. 1 with different embodiments of a label plate. The embodiments of Fig. 8 and 9 refer to a catheter and connector 31 as shown in Fig. 2 and 3 with further embodiments of a label plate.

The label plate 40 of Fig. 4 is quite similar to the label plate 10 depicted in Fig. 1 to 3 as it has similar first leg 41 and second leg 42 as well as protrusions 46 at the proximal end of legs 41, 42. However, web 43 connecting the first leg 41 and the second leg 43 is located at the lower side so that the handle-like section 3 is located between the legs 41, 42 on one side and the web 43 at the other side. Thereby the label plate 40 is clamped to the handle-like section 3 when the label plate 40 is fully mounted to the connector 1.

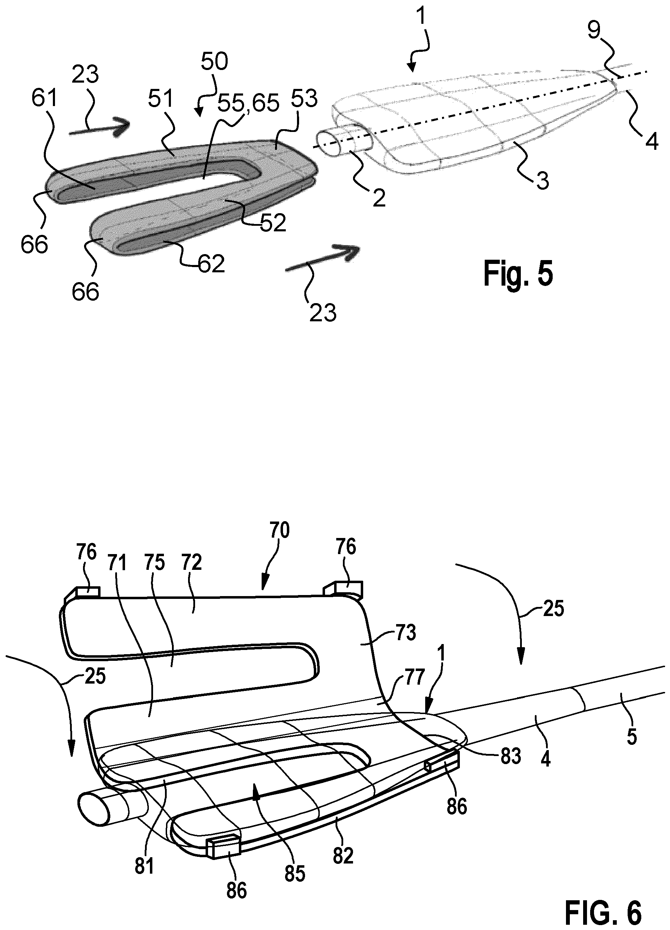

The label plate 50 shown in Fig. 5 comprises a first U-shaped segment comprising a first leg 51, a second leg 52 and a web 53 similar to the embodiment of Fig. 1 and an opposite, second U-shaped segment comprising a first leg 61, a second leg 62 and a web 63. Both segments form an intermediate space 55, 65 between legs 51, 52 and 61, 62, respectively. Both segments are connected by a bent transition section 66 at the distal end of the legs 51, 52, 61, 62, wherein the first leg 51 of the first segment is connected to the first leg 61 of the second segment and the second leg 52 of the first segment is connected to the second leg 62 of the second segment. For attachment to the connector 1 the label plate 50 is moved along the longitudinal axis in distal direction (see arrows 23). The connector 1 is clamped between the first and the second segments and by the transition section 66 so that the label plate 50 is thereby firmly fixed to the connector 1.

The label plate 70 depicted in Fig. 6 comprises two U-shaped segments which clamp the connector 1 therebetween, as well. Here, a first segment having a first leg 71, a second leg 72, an intermediate space 75 and a web 73 is folded along a hinge-like connection area 77 running parallel to the longitudinal axis 9 of the connector 1. The second segment has a first leg 81, a second leg 82, an intermediate space 85 and a web 83. The connection area 77 connects the first leg 71 along its full length (in longitudinal direction) to the first leg 81 of the second segment. During manufacturing, the label plate 70 is folded along the connection area 77 (see arrows 25) and firmly fixed to the connector 1 using two protrusions 76, 86 at each segment located at a rim of the segment opposite the connection area forming snap-fit connections.

The label plate 90 shown in Fig. 7 comprises initially two separate U-shaped segments each with a first leg 91, 101, a second leg 92, 102 and a web 93, 103 connecting the first leg 91, 101 to the second leg 92, 102. At each one of the four outer corners of the segments, the segments comprise a projection 96, 106 that is configured such that it forms a snap-fit connection with the opposite one 106, 96 when both segments are fully mounted to the connector 1. For that, each segment is moved towards the other one perpendicular to the longitudinal direction (see arrows 26, 27). In this embodiment, the connector 1 is located between the first segment and the second segment of the label plate 90, as well.

The label plate 110 (see Fig. 8) is similar to the embodiment shown in Fig. 7 since it has a first segment with a first leg 111, a second leg 112 and a web 113. The second segment has a first leg 121, a second leg and a web, as well. The label plate comprises a section 118 that increases the size of the area formed by the second leg 112. The section 118 may also be integrated to label plates of the embodiments shown in Figs. 1-6.

The similar embodiment of a label plate 110a depicted in Fig. 9 comprises a guide wire insertion tool 118a (instead of a section 118) that is provided on the rim of the second leg of this embodiment. This guide wire insertion tool 118a may be removed from the label plate 110a by breaking the plastic material at a pre-determined breaking point 119 at a web that connects the tool 118a to the second leg 112. Then, the tool 118a may be used as shown in Fig. 10 to 12 to insert a guide wire 130 into the distal end of the guide wire lumen. For that the distal tip 5c of the catheter is introduced into a through going passage of the tool 118 on one side (see Fig. 10) and the guide wire 130 on the other side (see Fig. 11). As the inner passage is reduced in its diameter at the central section of the tool 118a, the guide wire 130 is led to the opening of the distal tip 5c. This can be eased by slightly rotating the tool 118a (see arrow 30 in Fig. 12) so that the guide wire 130 is guided by the inner surface of the opening to the reduced diameter section.

The above description explains different embodiments of label plates which are cost-effective means for marking the connector/catheter, for example by laser printing or ink-jet printing.

## Claims

1. A label plate (10, 40, 50, 70, 90, 110, 110a) for a catheter connector (1, 31), wherein the label plate comprises a label plate body and at least one fixation element (16, 46, 66, 76, 86, 96, 106), wherein the label plate is configured to be fixed to an outer shell surface of the catheter connector by the at least one fixation element and wherein the shape of a bottom side or inner surface of at least a segment of the label plate body corresponds to the shape of an outer surface of at least a section of the catheter connector.

2. The label plate of claim 1, wherein the label plate is configured such that it allows exposure of a central portion (33a) of the catheter connector (1, 31) that extends along a longitudinal axis section of the catheter connector (1, 31).

3. The label plate of claim 1 or 2, wherein the at least one segment has a U-shape (11, 12, 13, 41, 42, 43, 51, 52, 53, 61, 62, 63, 71, 72, 73, 81, 62, 83, 91, 92, 93, 101, 102, 103, 111, 112, 113), wherein the U-shape provides an intermediate space (15, 45, 55, 65, 75, 85, 95, 105) to allow exposure of a central portion (33a) of the catheter connector that extends along the longitudinal axis section of the catheter connector.

4. The label plate according to claim 3, wherein the U-shaped segment is formed by at least one first plate-like leg (11, 41, 51, 61, 71, 81, 91, 101, 111, 121), at least one second plate-like leg (12, 42, 52, 62, 72, 82, 92, 102, 112) and at least one plate-like web (13, 43, 53, 63, 73, 83, 93, 103, 113) connecting one of the at least one first leg and one of the at least one second leg.

5. The label plate according to any one of the previous claims, wherein the at least one fixation element (16, 46, 66, 76, 86, 96, 106) and/or the label plate body is configured such that it provides a form-fitting connection, a force-fitting connection and/or a firmly bonded connection with the catheter connector.

6. The label plate according to any one of the previous claims, wherein a material located at the upper side or outer surface of the label plate body is writable or printable.

7. The label plate according to any one of the previous claims, wherein the label plate body comprises an appendage (118, 118a) providing a secondary function, wherein the appendage is, for example, formed as a guide wire insertion tool.

8. The label plate according to any one of the previous claims, wherein the label plate body comprises two opposite segments, wherein at least one of the two segments comprises the bottom side or inner surface with the shape which corresponds to the shape of the outer surface of at least a section of the catheter connector, wherein both segments are connected by a hinge (77).

9. The label plate according to any one of the previous claims, wherein the at least one fixation element (16, 46, 66, 76, 86, 96, 106) and/or the label plate body comprises a predetermined breaking point (119).

10. The label plate according to any one of the previous claims, wherein an RFID tag is embedded within the label plate body material.

11. System comprising a catheter connector (1, 31) and a label plate (10, 40, 50, 70, 90, 110, 110a) according to any one of the previous claims.

12. System comprising a catheter having an elongated, flexible shaft (5) and a connector (1, 31) at the proximal end of the shaft (5), wherein the system further comprises a label plate (10, 40, 50, 70, 90, 110, 110a) according to any one of the claims 1 to 10.

13. The system of any one of the claims 11 to 12, wherein the catheter connector section (2, 32) of the connector (1, 31) is a Luer connector.

14. The system of any one of the claims 12 to 13, wherein the catheter comprises an inflatable balloon (7) at the distal end of the catheter shaft (5).
